# EUROPEAN PATENT SPECIFICATION

(11) **EP 0 928 781 B1**
(45) Date of publication and mention of the grant of the patent: **27.03.2002**
(21) Application number: 98124607.7
(22) Date of filing: 23.12.1998
(51) Int. Cl.: C07C 51/27, C07C 66/02, C07C 67/08, C07C 69/16

(54) **Process for the preparation of rhein and its diacyl derivatives**
Verfahren zur Herstellung von Rhein und seinen Diacylderivaten
Procédé de préparation de rhéine et de ses dérivés diacylés

(30) Priority: 30.12.1997 IT MI972899
(43) Date of publication of application: 14.07.1999
(73) Proprietor: LABORATOIRE MEDIDOM S.A., 1211 Genève 12 (CH)
(72) Inventor: Di Napoli, Guido, 1245 Collonges-Bellerive (CH)
(74) Representative: Gervasi, Gemma, Dr.

(56) References cited:
- EP-A- 0 636 602
- US-A- 3 678 107
- US-A- 4 013 680
- ZEMBOWER D.E. ET AL.: "NOVEL ANTHRAQUINONE INHIBITORS OF HUMAN LEUKOCYTE ELASTASE AND CATHEPSIN G1" JOURNAL OF MEDICINAL CHEMISTRY, vol. 35, 1992, pages 1597-1605, XP002069744
- MATT C., WAGNER A., MIOSKOWSKI, C.: "NOVEL TRANSFORMATION OF PRIMARY NIROALKANES AND PRIMARY ALKYL BROMIDES TO THE CORRESPONDING CARBOXYLIC ACIDS" JOURNAL OF ORGANIC CHEMISTRY, vol. 62, 1997, pages 234-235, XP002069745

## Description

The present invention regards a process for the preparation of rhein and corresponding diacyl derivatives, in particular diacerein, via oxidation of aloe-emodin or its triacyl derivatives, such as triacetyl aloe-emodin, with salts of nitrous acid, possibly followed by acylation of the rhein thus obtained.

Rhein and various 1,8-diacyl derivatives, among which diacerein (GB 1.578.452), are known for their therapeutic properties in the treatment of degenerative disorders of articulations and/or of the connective tissue, such as osteoporosis and rheumatoid arthritis, and in long-term treatment of osteo-arthritis (Malterud *et al*., "Antioxidant and Radical Scavenging Effects of Anthraquinones and Anthrones", Pharmacol., (Basel), 47 Suppl. 1; 77-85, 1993).

Rhein moreover is of particular interest for other pharmacological activities, such as bacteriostatic activity (Wang *et al*., Biochemical Study of Chinese Rhubarb - Inhibition of Anthraquinone Derivatives on Anaerobic Bacteria; "Zhongguo Yaoke Daxue Xuebao"; 21(6), 354-57, 1990), or anti-carcinogenic activity, for example in association with adriamycin (Fanciulli *et al*., "Inhibition of Membrane Redox Activity by Rhein and Adriamycin in Human Glioma Cells", Anti-carcinogenic drugs, 3(6), 615-21, 1992).

### State of the art

Various examples are known of preparation of anthraquinonic acids, which use hexavalent-chromium oxidants or manganese oxidants, among which those described by H. Nawa, M. Uchibayashi and T. Masuka (J. Org. Chem., 26, 979, 1961) and by W.M. Owton, M. Brunavs, M.V. Miles, D.R. Dobson and D.J. Steggles (J. Chem. Soc., Perkin I, 931, 1995).

The preparation of diacerein is known via oxidation with hexavalent chromium of aloe-emodin ("Sostanze farmaceutiche", Italian translation and review by R. Longo, OEMF, Milan, 1988, p. 596, of "Pharmazeutische Wirkstoffe, Synthesen, Patente, Anwedungen", George Thieme Verlag, Stuttgart-New York, 1982-1987) or of triacyl aloe-emodin, in turn obtained by acetylation of aloe-emodin (H. Nawa *et al*., see above).

The only process of synthesis of diacerein actually used on an industrial scale involves acetylation of aloin, followed by oxidation with chromic anhydride (EP-A-636.602, in the name of the Applicant).

### Technical problem

As far as chromium oxidants are concerned, their industrial use poses various problems of environmental impact, connected with the disposal of the corresponding waste.

The exhausted reaction waters and the hydro-organic washing phases in fact contain salts of these metals, which are toxic for human health and dangerous for the environment.

As regards chromium salts, see, for example, what is stated by the European Community, according to which chromium anhydride "may cause cancer by inhalation" and defines potassium dichromate as "irritating for the eyes, the respiratory tract and the skin" (See EEC Directive 94/54/CE of July 30, 1996).

### Summary

Now the Applicant has found that the technical problem referred to above is overcome by performing the oxidation of aloe-emodin or triacetyl aloe-emodin by means of a nitrite in an acid environment.

The Applicant has in particular found a process for the preparation of rhein and rhein derivatives of formula (I) 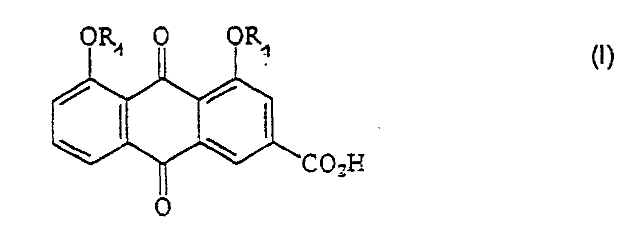 in which R₁ is H or an acyl,
comprising the oxidation of a derivative of formula (II) 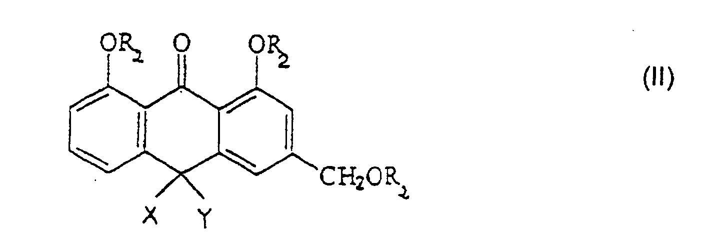 in which R₂ is H or an aliphatic acyl, the same as or other than R₁,
and X and Y, taken together, are an =O group, or X is H and Y is the group A, as shown below: 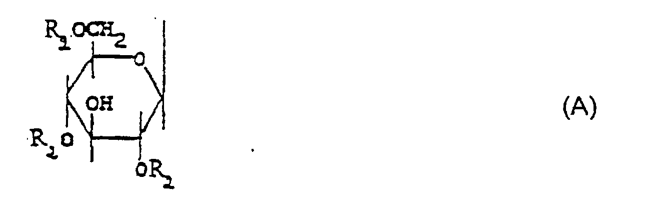 in which R₂ is as defined above,
with a salt of nitrous acid, in an acid environment,
to obtain rhein of formula (la) [compound of formula (I) in which R₁ = H] 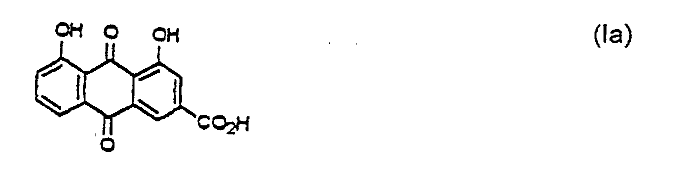 possibly followed by acylation of the rhein thus obtained when the aim is to obtain the derivatives of formula (I) in which R₁ is an acyl.

The use of sodium nitrite for the conversion of alcohols into acids is not known to the prior art (this methodology is not described, for example, by J. March, in Advanced Organic Chemistry, 3rd Ed., 1985, J. Wiley & Sons, page 1084, section 9.22 on oxidation of alcohols to carboxyl groups).

Oxidation with a nitrite is extremely advantageous if compared with that obtained using chromium oxidants, the residues of which must be stored in special dumps for toxic or harmful waste, whereas the exhausted waters coming from the present process can be easily treated with the normal methods used for disposal of nitrites and nitrates.

The present process is in particular suited for obtaining diacerein [compound of formula (I) in which R₁ is an acetyl].

### Detailed description of the invention

In the present text, the acyl group R₁ of the derivatives of formula (I) is typically an R_{A}-CO- group, where R_{A} is an aliphatic or aromatic group having from 1 to 12 carbon atoms, more in particular an alkyl having from 1 to 3 carbon atoms, the said group being either linear or branched, possibly substituted with one or more halogen atoms. For example, R₁ may be an acetyl, trifluoro-acetyl, trichloro-acetyl, more in particular an acetyl.

The group R₂ is H or an R_{B}-CO- aliphatic acyl, where R_{B} is an alkyl group preferably having from 1 to 3 carbon atoms, the said group being linear or branched, possibly substituted with one or more halogen atoms. For example, R₂ may be an acetyl, trifluoro-acetyl, trichloro-acetyl, more in particular an acetyl.

According to a preferred embodiment of the present invention, the substrate to be oxidized is the derivative of formula (II) in which X and Y, taken together are an =O group, and more in particular, R₂ is H (aloe-emodin) or an acetyl (triacetyl aloe-emodin).

Alternatively, the oxidation may, for example, be performed on the substrate of formula (II) in which X is H and Y is A, in which R₂ = H (aloin).

The oxidation reaction according to the present process may be conducted using various salts of nitrous acid, in particular inorganic salts, such as salts of alkaline or alkaline-earth metals, as for instance sodium nitrite, potassium nitrite, or calcium nitrite. Typically, sodium nitrite is used.

Preferably the salt of nitrous acid is used in molar excess with respect to the substrate to be oxidized, for example 2 to 50 mol., preferably from 8 to 15 mol. of nitrite per mole of derivative of formula (II).

The reaction medium used for the present oxidation with nitrite is preferably an acid, for example, a mineral acid, in particular a strong mineral acid, such as sulphuric acid, or else an organic acid, for instance a carboxylic acid, possibly halogenated, such as acetic acid, trifluoro-acetic acid, trichloro-acetic acid, or else a sulphonic acid, such as methanesulphonic acid.

The mineral acid is, for example, concentrated sulphuric acid (H₂SO₄ with a commercial concentration of 95-98%), or else aqueous H₂SO₄, in particular with a concentration of 50% v/v.

The acid may possibly be used in the presence of a co-solvent, for example water or an aprotic polar solvent, such as dimethyl sulphoxide, acetone, or acetonitrile.

Preferably, the present oxidation is conducted in a mineral. acid used as reaction medium, possibly in the presence of water, more preferably in concentrated sulphuric acid or in methanesulphonic acid.

The quantity of solvent (for example, acid, possibly mixed with a co-solvent) used as reaction medium in the present oxidation is, for example, of from 10 to 100 ml, in particular of from 15 to 50 ml, per gram of derivative of formula (II).

According to an even more preferred embodiment of the present invention, the oxidation of the derivatives of formula (II) is carried out in the presence of boric acid or of one of its salts, for example an inorganic salt, such as sodium tetraborate, preferably used in molar excess with respect to the substrate to be oxidized, for example from 10 to 40 mol., more preferably from 15 to 30 mol. of boric acid or corresponding salt per mole of derivative of formula (II).

The present oxidation is performed at temperatures generally ranging from room temperature (approximately from +20°C to +25°C) to the boiling temperature of the reaction mixture, preferably of between +50°C and +130°C, more typically of between +100°C and +120°C, and takes place in a period generally lasting from a few hours to 48 hours.

According to a typical embodiment of the present invention, the reaction is carried out in concentrated sulphuric acid or in methanesulphonic acid, used as reaction medium, in the presence of boric acid, at a temperature of between +100°C and +120°C.

The present process is moreover advantageous in so far as it is suitable for oxidizing aloe-emodin or triacetyl aloe-emodin also in the crude state, for example having a titre of 70-90%, containing the by-products that are present together with aloe-emodin in the process of extraction from vegetal sources or in semi-synthetic preparative processes known in the literature.

The aloe-emodin may be the one available on the market or may be prepared as described in the literature, for example by M. Rychener *et al.* (Pharm. Acta Helv., 64, 8, 1989).

Triacetyl aloe-emodin may be obtained by acetylation of aloe-emodin with acetic anhydride, preferably as reaction solvent, in the presence of catalytic quantities of an acid or a base (for example, H₂SO₄ or sodium acetate), operating, for example, at a temperature of between room temperature and the temperature of reflux of the reaction mixture, or according to other conventional methodologies.

Chen When-Hao *et al*. (Journal of Nanjing College of Pharmacy, 1986, 17(1), 1-4; Chemical Abstract, Vol. 105, 1986, 105:226138z) describes the preparation of aloe-emodin by treatment of aloin with FeCl₃, followed by acetylation of aloe-emodin to triacetyl aloe-emodin.

According to a particular embodiment of the present invention, aloe-emodin is obtained by treatment of aloin with a ferric acid, such as FeCl₃, in an acid environment, for example in aqueous hydrochloric acid, for example at a temperature of approximately from +90°C to +110°C. Generally a molar excess of ferric salt is used with respect to the aloin, for example from 5 to 15 mol., more in particular 10 mol., per mole of aloin. The reaction environment may in particular consist of HCI 0.5N, for example in the quantity of approximately 10 ml per gram of aloin.

For the purposes of the present invention, the crude aloin thus obtained may undergo acylation directly; for example it may be acetylated to triacetyl aloe-emodin.

According to a particular embodiment of the present invention, aloe-emodin is converted into triacetyl aloe-emodin by treatment with acetic anhydride, preferably as reaction solvent, in the presence of an acidic or basic catalyst (such as concentrated H₂SO₄ or sodium acetate), operating at a temperature generally ranging from room temperature (i.e., approx. from +20°C to +25°C) to the reflux temperature, for example from +90°C to +100°C.

In the preparation of triacetyl aloe-emodin, for example from 3 to 15 ml of acetic anhydride are used per gram of product, calculated on crude or pure aloe-emodin, for example 10 ml per gram of pure aloe-emodin. The catalyst, for example sodium acetate, is used in a quantity of from 10 wt% to 30 wt% with respect to the crude or pure aloe-emodin, for example 30 wt% with respect to the aloe-emodin content.

According to a particular embodiment of the present invention, crude triacetyl aloe-emodin is purified by crystallisation using toluene, possibly mixed with methanol, and heating until substantial dissolution is achieved (for example, up to a temperature of between +60°C and the reflux temperature of the reaction mixture) and cooling until precipitation. For example, a first crystallisation using toluene is carried out (reflux heating, and then cooling until precipitation), followed by a second crystallisation using a toluene-methanol mixture, heating to a temperature of from +60°C to +70°C, and then cooling down to a temperature of from +3°C to +5°C.

The acylation of rhein to 1,8-diacyl derivatives of formula (II) may be performed by treatment with acylating agents adopting to conventional techniques, for example by treatment with an anhydride or with the acyl halide of the acid corresponding to the acyl group that it is intended to introduce (for example, the acid R_{A}COOH, where R_{A} is as defined above). Typically the halide is used in the presence of a basic catalyst, and the anhydrides in the presence of an acidic or basic catalyst.

The acidic catalyst may, for example, be acetic acid, methanesulphonic acid, trifluoromethanesulphonic acid, concentrated sulphuric acid, and the basic catalyst may be sodium acetate or NaCO₃.

The acylation of rhein is carried out at temperatures preferably of between room temperature (+20-25°C) and +100°C.

According to a particular embodiment of the present invention, rhein is converted into diacerein via acetylation, preferably via treatment with acetic anhydride, in molar excess with respect to rhein. Preferably, rhein is treated with acetic anhydride, preferably used as reaction medium, in the presence of a catalytic quantity of concentrated H₂SO₄, preferably operating at a temperature ranging from room temperature (approx. +20-25°C) to the reflux temperature, for example +130-133°C.

Diacerein is easily isolated from the reaction medium in so far as it precipitates by addition of water, and is then separated using conventional means, such as filtration, and dried.

The other diacyl derivatives of formula (I) may be isolated according to conventional techniques.

The oxidation products of formula (II) and the end products of formula (I) may be easily purified according to the usual techniques of preparative organic chemistry.

In particular, diacerein may be purified via one or more crystallisation operations using a solvent chosen from between 2-methoxyethanol and *N,N*-dimethylacetamide, for example via three successive crystallisation operations using *N*,*N*-dimethylacetamide, preferably in the presence of acetic anhydride, heating the product to be purified in the crystallisation solvent until substantially complete dissolution is achieved, and then leaving to cool down until precipitation, according to the method described in EP-A-636.602 and in the corresponding patent USP 5.670.695, the content of which is incorporated in the present patent application for reference.

According to a particular embodiment of the present invention, diacerein is purified by crystallisation using *N*,*N*-dimethylacetamide (in a quantity, for example, of approx. 4 vol. per gram of crude product), in the presence of acetic anhydride (in a quantity, for example of 0.5-1.0 vol.% with respect to the dimethylacetamide), for example heating to +100°C, and then cooling down to +3-5°C.

Crystallisation using *N*,*N*-dimethylacetamide is preferably followed by the conversion of diacerein into the corresponding alkaline metal salt (e.g., potassium salt), for example by treatment with potassium acetate, typically in molar excess, in a acetone-water mixture. The diacerein is then released from its salt by acidification, for example using sulphuric acid (such as 10% H₂SO₄) in a mixture of water and organic solvent mixable with water, such as a water-ethanol mixture.

It is important to point out that the crude diacerein obtained from the present process contains very low amounts of aloe-emodin, i.e., lower than 50 ppm, which, after purification, drop to values of less than 4 ppm.

Below are given a number of examples with the purpose of illustrating the present invention, without, however, limiting in any way the scope thereof.

### Example 1

To a mixture of aloe-emodin (10 g; HPLC titre, 78.7%) and boric acid (40 g) in concentrated sulphuric acid (150 ml), sodium nitrite (25.6 g) is added, and then the mixture is heated to +120°C for 1.5 hours. The reaction mixture is then poured into water and ice (200 ml), and the precipitate that has formed is filtered and dried in an oven at a temperature of +50°C to obtain 8.9 g of rhein (yield, 74%; HPLC titre, 69%).
IR (nujol): 3100-3000, 1692, 1631 cm⁻¹
¹H-NMR (DMSO-d₆): delta 7.40 (1H, d); 7.76 (1H, d); 7.83 (1H, t); 8.14 (1H, d); 11.88 (2H, br); 13.5 (1H, br).

### Example 2

A mixture of aloe-emodin (19 g; HPLC titre, 78.7%), sodium nitrite (41.5 g) and methanesulphonic acid (450 ml) is heated slowly to +100°C for 3 hours. The reaction mixture is then poured into water and ice (1350 ml), and the precipitate that has formed is filtered, washed, and vacuum-dried at +60°C to obtain 17.4 g of rhein (yield, 74%; HPLC titre, 68.1%), identified as such in so far as it has the chemical-physical characteristics of the product of Example 1.

### Example 3

A mixture of triacetyl aloe-emodin (25 g; HPLC titre, 72%), sodium nitrite (41.5 g), boric acid (65 g), and sulphuric acid (1230 ml) is slowly heated up to +110°C for 5 hours. The reaction mixture is then poured into water and ice (3530 ml), and the precipitate that has formed is filtered, washed, and vacuum-dried in an oven at +50°C until constant weight is reached, to obtain 13 g of rhein (yield, 73%; HPLC titre, 72%), identified on the basis of the chemical-physical characteristics, which are found to be the same as those of the product of Example 1.

### Example 4

A mixture of triacetyl aloe-emodin (25 g; HPLC titre, 88.4%), sodium nitrite (41.5 g), boric acid (65 g), and methanesulphonic acid (530 ml) is kept stirred at room temperature for 4 hours, and then heated to +110°C for 18 hours. The reaction mixture is poured into water (1450 ml), and the precipitate that has formed is filtered, washed, and vacuum-dried until constant weight is reached, to obtain 14.5 g of rhein (yield, 81%; HPLC titre, 89%), identified on the basis of the chemical-physical characteristics, which are found to be the same as those of the product of Example 1.

### Example 5

A mixture of triacetyl aloe-emodin (25 g; HPLC titre, 88.4%), sodium nitrite (41.5 g), and methanesulphonic acid (530 ml) is heated up +110°C for 2 hours. The reaction mixture is poured into water (1500 ml), and the precipitate that has formed is filtered, washed, and vacuum-dried until constant weight is reached, to obtain 14 g of rhein (yield, 77%; HPLC titre, 87%), identified on the basis of the chemical-physical characteristics, which are found to be the same as those of the product of Example 1.

### Example 6

To a mixture of rhein (2 g) in acetic anhydride (14 ml), cooled down to 0°C, is added sulphuric acid (0.14 ml). The mixture is then brought up to room temperature and kept at this temperature for 6 hours. The reaction mixture is poured into water (50 ml), and the precipitate that has formed is filtered and dried in an oven until constant weight is reached.
IR (nujol): 3100-3000, 1767, 1690-1680 cm⁻¹
¹H-NMR (DMSO-d₆): delta 2.39 (6H, s); 7.64 (1H, d); 7.95 (1H; d); 8.03 (1H, d); 8.14 (1H, d); 8.55 (1H, d); 13.85 (1H, br).

### Example 7 - Preparation of diacerein from aloin

### A) Conversion of aloin into aloe-emodin

A mixture of aloin (150 g) and HCI 0.5N (750 ml) is reflux heated; then a solution previously prepared and filtered on celite, consisting of iron trichloride hexahydrate (969 g) and hydrochloric acid 0.5N (750 ml) is added drop by drop in approximately 60-90 minutes, proceeding with reflux heating (at approx. +103°C) for a further 7 hours.

The mixture is cooled down to +3-5°C and vacuum filtered. The panel is washed with demineralized water until the mother liquor is practically colourless. The product is vacuum dried at +70°C until constant weight is reached, to obtain 109 g of a solid having an HPLC titre of 48.26% (yield, 54%).

### B) Conversion of aloe-emodin into triacetyl aloe-emodin

A mixture of the aloe-emodin coming from the previous step (109.0 g, HPLC titre 48.26%, containing 52.6 g of aloe-emodin), acetic anhydride (545 ml; in a ratio 1:5 with respect to aloe-emodin as such) and sodium acetate (16.33 g; 15 wt% with respect to aloe-emodin as such) is heated up to +100°C and kept at this temperature for approximately 3.5-4 hours. The mixture is then cooled down to approximately +50°C and vacuum concentrated until a semisolid consistency is obtained. Toluene (200 ml) is added, and the mixture is re-concentrated until dry. The solid thus obtained is crystallised as follows: toluene is added (2178 ml; in a ratio of 1:20 with respect to aloe-emodin as such) together with ENO carbon (11 g). The product is reflux heated (approx. 1 hour), cooled down to +40°C, and the suspension is filtered on a celite bed, heat washing with toluene (1089 ml), and the filtered solution is vacuum concentrated until a dense paste is obtained.

A semi-solid crude product is obtained, which is re-crystallised by addition of toluene (250.5 ml) and methanol (326.7 ml), heating until complete solution is achieved (approx. +60-70°C). The product is cooled down to +3-5°C and kept at this temperature for approximately 1 hour. It is then vacuum filtered, and the panel is washed with methanol (110 ml) until the mother liquor is colourless.

The product is vacuum dried to constant weight to obtain 59 g of product having an HPLC titre of 90.73% and a yield of 69.40%.

(The crystallisation mother liquor contains a further 9.70 g of triacetyl aloe-emodin).

### C) Conversion of triacetyl aloe-emodin into rhein

Triacetyl aloe-emodin (59.0 g, HPLC titre 90.73%, containing 53.5 g of 100% pure product), boric acid (173.5 g) and, in portions, sodium nitrite (109.7 g) are added to methanesulphonic acid (1413 ml) which has been cooled down to +10-12°C.

The addition of sodium nitrite is exothermic (i.e., the temperature rises from +14°C to +42°C). The product is kept stirred at +40°C (spontaneous) for approximately 4 hours, and then heated to +105°C (±5°C) for 18-20 hours. An HPLC check is carried out to determine the degree of conversion and the absence of an intermediate product. The reaction mass is cooled down to +80°C and poured into water having a temperature of +5°C (3845 ml; in a ratio of 1:2.72 with respect to the methanesulphonic acid). It is then cooled down to +40°C and vacuum filtered.

It is washed with water until a neutral pH is achieved, and vacuum dried at +60°C, to obtain 41.3 g of product, having an HPLC titre of 91% corresponding to a yield of 98%.

### D) Conversion of rhein into diacerein

A mixture of rhein (HPLC titre, 91%; 41.3 g), acetic anhydride (413.0 ml; in a ratio of 1:10 with respect to rhein) and 98% sulphuric acid (4.13 ml) is heated to +130-133°C and kept at this temperature for 30 minutes. The mixture is cooled down to approximately +80°C, and acetic acid is added (82.6 ml; in a ratio of 1:2 with respect to rhein). It is then cooled down to +20°C and vacuum filtered. The panel is washed 3 times with acetic acid (with 16.5, 16.5 and 41.3 ml, respectively), and finally with water until a neutral pH is achieved. It is then vacuum dried at +60-70°C, to obtain 50.6 g of product having an HPLC titre of 96.44% corresponding to a yield of 99%. The aloe-emodin content of the crude product thus obtained is approximately 31.7 ppm.

### E) Purification of diacerein

A mixture of crude diacerein (50.6 g), dimethylacetamide (206.0 ml) and acetic anhydride (1.49 ml) is heated to +100°C and kept at this temperature for approximately 1 hour, and the dissolution is monitored until complete. The product is cooled down to a temperature of between 0 and +3°C, and filtered. The panel is washed with acetone, to obtain 81 g of wet solid.

### Salification of diacerein

Acetone (506 ml)is added to the wet solid (81 g), and a solution previously prepared, consisting of potassium acetate (25.3 g) in water (25.3 g) is added drop by drop, keeping the temperature at +20-25°C. A very dense suspension is obtained, which is reflux heated at approximately +57°C for 2 hours. The suspension fluidifies. It is then cooled down to +3°C for approximately 1 hour and vacuum filtered. The panel is washed with acetone and vacuum dried at +90°C, to obtain 63.4 g of potassium salt of diacerein.

### Release of diacerein from its salt

A mixture of dry potassium salt of diacerein (63.4 g), absolute ethanol (632.5 ml) and demineralized water (632.5 ml) is kept at +20-22°C until complete dissolution.

SA 189 carbon is added (5.0 g), and after approximately 1 hour the product is filtered on a celite bed, washing with an ethanol-water mixture. The diacerein is precipitated by adding to the filtered solution 10% sulphuric acid until pH 4.0 is reached. It is then vacuum filtered, and the panel is washed until the sulphates disappear. Finally, it is vacuum dried to constant weight, to obtain 43.9 g of product having an HPLC titre of 99%.

### Example 8 - Preparation of diacerein by direct oxidation of aloin

In cold conditions, aloin (1.1 g; 2.63 mmol.) is dissolved in concentrated sulphuric acid. Boric acid (0.8 g; 12.9 mmol.) and sodium nitrite (1.8 g; 26.08 mmol.) are added, and the temperature is brought up to +120°C. After 2.5 hours, the reaction mixture is poured into 60 ml of ice, washing with water (10 ml). The mixture is filtered on a Buchner's tube and the product is dried in an oven, to obtain 1.2 g of crude product.

## Claims

1. Process for the preparation of rhein or rhein derivatives of formula (I) 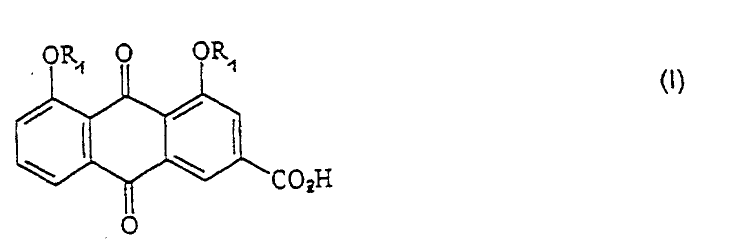 in which R₁ is H or an acyl,
comprising the oxidation of a derivative of formula (II) 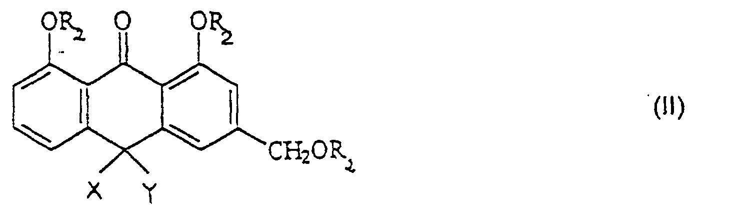 in which R₂ is H or an aliphatic acyl, the same as or other than R₁,
and X and Y, taken together, are an =O group, or X is H and Y is the group A, as shown below: 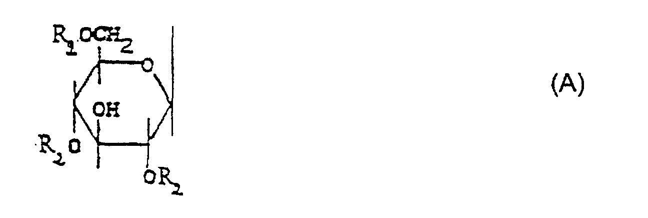 in which R₂ is as defined above,
with a salt of nitrous acid, in an acid environment,
to obtain rhein of formula (la) 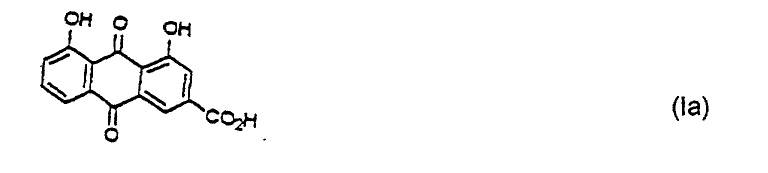 possibly followed by acylation of the rhein thus obtained when the aim is to obtain the derivatives of formula (I) in which R₁ is an acyl.

2. Process according to Claim 1, in which the salt of nitrous acid is sodium nitrite.

3. Process according to Claim 1, in which the salt of nitrous acid is used in molar excess with respect to the derivative of formula (II).

4. Process according to Claim 1, in which the salt of nitrous acid is used in a quantity of from 8 to 15 mol. of nitrite per mole of derivative of formula (II).

5. Process according to Claim 1, in which the said oxidation is carried out in a mineral acid or in an organic acid as reaction medium.

6. Process according to Claim 5, in which the acid is chosen from between concentrated sulphuric acid and methanesulphonic acid.

7. Process according to Claim 1, in which the said oxidation is conducted in the presence of boric acid or of one of its salts.

8. Process according to Claim 7, in which the boric acid or corresponding salt is used in molar excess of between 15 and 30 mol. per mole of derivative of formula (II).

9. Process according to Claim 1, in which the oxidation is carried out at a temperature of between +20°C and the boiling temperature of the reaction mixture.

10. Process according to Claim 1, in which the oxidation is conducted at a temperature of between +50°C and +130°C.

11. Process according to Claim 1, in which the derivative of formula (II) is chosen from between aloe-emodin and triacetyl aloe-emodin.

12. Process according to Claim 1, in which the said oxidation is carried out in concentrated sulphuric acid or in methanesulphonic acid, used as reaction medium, in the presence of boric acid, at a temperature of between +100°C and +120°C.

13. Process according to Claim 1, comprising an acetylation phase which yields diacerein (compound of formula (I) in which R₁ is an acetyl).

14. Process according to Claim 13, further comprising at least one operation of crystallisation of diacerein using a solvent chosen from between 2-methoxyethanol and *N,N*-dimethyl acetamide.

15. Process according to Claim 1, further comprising the preparation of aloe-emodin by treatment of aloin with a ferric salt, in an acid environment.

16. Process according to Claim 1, moreover comprising the preparation of triacetyl aloe-emodin by conversion of aloin into aloe-emodin by treatment with a ferric salt, in an acid environment, followed by acetylation with acetic anhydride, in the presence of sulphuric acid as catalyst, followed by crystallisation of triacetyl aloe-emodin using toluene, possibly in a mixture with methanol.

## Patentansprüche

1. Verfahren zur Herstellung von Rhein oder Rhein-Derivaten der Formel (I) 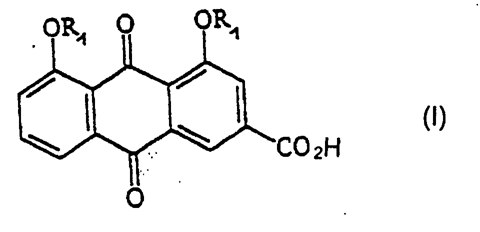 worin R₁ für H oder Acyl steht,
das umfasst die Oxidation eines Derivats der Formel (II) 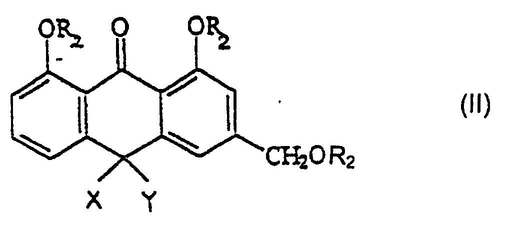 worin R₂ für H oder ein aliphatisches Acyl, das mit R₁ identisch oder davon verschieden ist, steht und X und Y gemeinsam eine =O-Gruppe darstellen oder X für H und Y für die Gruppe A der nachstehenden Formel stehen 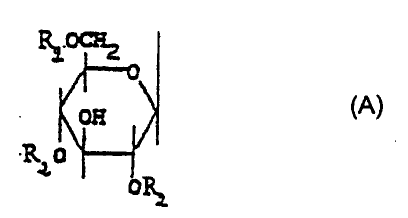 worin R₂ wie oben definiert ist,
mit einem Salpetrigsäuresalz in einer Säure-Umgebung zur Herstellung von Rhein der Formel (la) 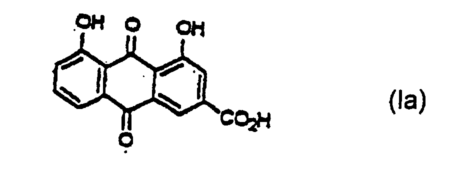 woran sich möglicherweise die Acylierung des so erhaltenen Rheins anschließt, wenn Derivate der Formel (I) hergestellt werden sollen, in denen R₁ für Acyl steht.

2. Verfahren nach Anspruch 1, worin das Salpetrigsäuresalz Natriumnitrit ist.

3. Verfahren nach Anspruch 1, worin das Salpetrigsäuresalz in molarem Überschuss gegenüber dem Derivat der Formel (II) verwendet wird.

4. Verfahren nach Anspruch 1, worin das Salpetrigsäuresalz in einer Menge von 8 bis 15 mol Nitrit pro mol Derivat der Formel (II) verwendet wird.

5. Verfahren nach Anspruch 1, worin die genannte Oxidation in einer Mineralsäure oder in einer organischen Säure als Reaktionsmedium durchgeführt wird.

6. Verfahren nach Anspruch 5, worin die Säure ausgewählt wird aus der Gruppe konzentrierte Schwefelsäure und Methansulfonsäure.

7. Verfahren nach Anspruch 1, worin die genannte Oxidation in Gegenwart von Borsäure oder einem ihrer Salze durchgeführt wird.

8. Verfahren nach Anspruch 7, worin die Borsäure oder ihr entsprechendes Salz in einem molaren Überschuss zwischen 15 und 30 mol pro mol Derivat der Formel (II) verwendet wird.

9. Verfahren nach Anspruch 1, worin die Oxidation bei einer Temperatur zwischen +20°C und der Siedetemperatur der Reaktionsmischung durchgeführt wird.

10. Verfahren nach Anspruch 1, worin die Oxidation bei einer Temperatur zwischen +50 und +130°C durchgeführt wird.

11. Verfahren nach Anspruch 1, worin das Derivat der Formel (II) ausgewählt aus Aloe-emodin und Triacetyl-aloe-emodin.

12. Verfahren nach Anspruch 1, worin die genannte Oxidation in konzentrierter Schwefelsäure oder in Methansulfonsäure, die als Reaktionsmedium verwendet wird, in Gegenwart von Borsäure bei einer Temperatur zwischen +100 und +120°C durchgeführt wird.

13. Verfahren nach Anspruch 1, das eine Acetylierungsphase umfasst, die Diacerhein (eine Verbindung der Formel (I), worin R₁ für Acetyl steht) ergibt.

14. Verfahren nach Anspruch 13, die außerdem mindestens eine Kristallisation von Diacerhein umfasst, bei der ein Lösungsmittel aus der Gruppe 2-Methoxyethanol und N,N-Dimethylacetamid verwendet wird.

15. Verfahren nach Anspruch 1, das außerdem die Herstellung von Aloe-emodin durch Behandlung von Aloin mit einem Eisen(III)salz in einer Säure-Umgebung umfasst.

16. Verfahren nach Anspruch 1, das außerdem die Herstellung von Triacetyl-aloe-emodin umfasst durch Umwandlung von Aloin in Aloe-emodin durch Behandeln mit einem Eisen(III)salz in einer Säureumgebung und anschließende Acetylierung mit Essigsäureanhydrid in Gegenwart von Schwefelsäure als Katalysator, woran sich eine Kristallisaton des Triacetyl-aloe-emodins unter Verwendung von Toluol, möglicherweise im Gemisch mit Methanol, anschließt.

## Revendications

1. Procédé pour la préparation de rhéine ou de dérivés de rhéine de la formule (1) 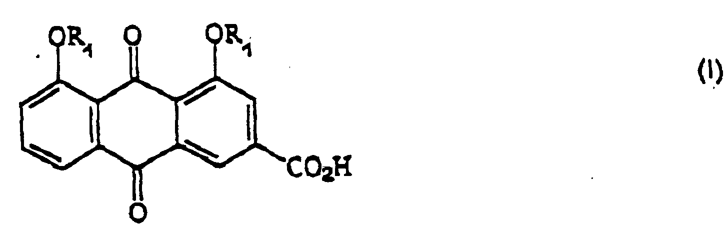 dans laquelle R₁ est H ou un acyle,
comprenant l'oxydation d'un dérivé de formule (II) 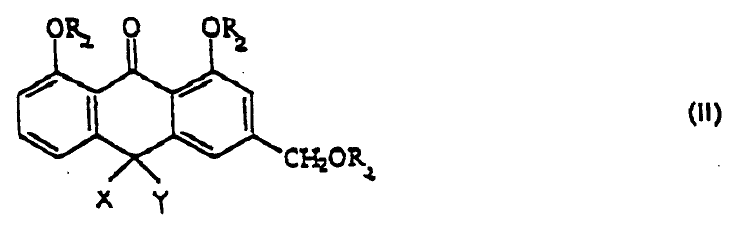 dans laquelle R₂ est H ou un acyle aliphatique, le même que ou autre que R₁,
et X et Y, pris ensemble, sont un groupe =O, ou bien X est H et Y est le groupe A, comme montré ci-dessous: 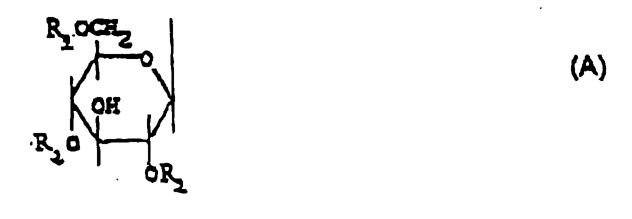 où R₂ est tel que défini ci-dessus,
avec un sel d'acide nitreux dans un environnement acide, pour obtenir la rhéine de la formule (Ia) 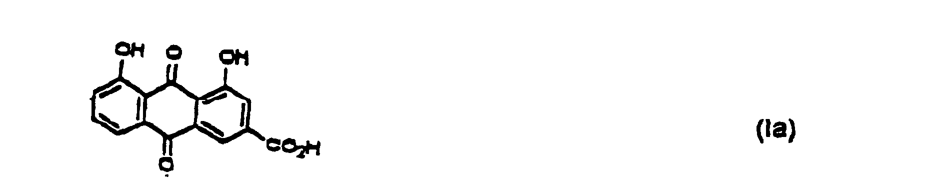 avec éventuellement ensuite l'acylation de la rhéine ainsi obtenue quand le but est d'obtenir des dérivés de formule (I) dans laquelle R₁ est un acyle.

2. Procédé selon la revendication 1, où le sel d'acide nitreux est le nitrite de sodium.

3. Procédé selon la revendication 1, où le sel d'acide nitreux est utilisé dans un excès molaire par rapport au dérivé de formule (II).

4. Procédé selon la revendication 1, où le sel d'acide nitreux est utilisé en une quantité de 8 à 15 moles de nitrite par mole du dérivé de formule (II).

5. Procédé selon la revendication 1, où ladite oxydation est effectuée dans un acide minéral ou dans un acide organique en tant que milieu réactionnel.

6. Procédé selon la revendication 5, où l'acide est choisi entre de l'acide sulfurique concentré et de l'acide méthanesulfonique.

7. Procédé selon la revendication 1, où ladite oxydation est entreprise en présence d'acide borique ou de l'un de ses sels.

8. Procédé selon la revendication 7, où l'acide borique ou sel correspondant est utilisé en excès molaire entre 15 et 30 moles par mole du dérivé de formule (II).

9. Procédé selon la revendication 1, où l'oxydation est effectuée à une température entre +20°C et la température d'ébullition du mélange réactionnel.

10. Procédé selon la revendication 1, où l'oxydation est entreprise à une température entre +50°C et +130°C.

11. Procédé selon la revendication 1, où le dérivé de formule (II) est choisi entre l'aloe-émodine et la triacétyl aloé-émodine.

12. Procédé selon la revendication 1, où ladite oxydation est effectuée dans de l'acide sulfurique concentré ou de l'acide méthanesulfonique, utilisé en tant que milieu réactionnel, en présence d'acide borique, à une température entre +100°C et +120°C.

13. Procédé selon la revendication 1, comprenant une phase d'acétylation qui donne la diacéréine (composé de formule (I) où R₁ est un acétyle).

14. Procédé selon la revendication 13, comprenant de plus au moins une opération de cristallisation de la diacéréine en utilisant un solvant choisi entre le 2-méthoxyéthanol et le *N,N*-diméthyl acétamide.

15. Procédé selon la revendication 1, comprenant de plus la préparation d'aloé-émodine par traitement de l'aloïne avec un sel ferrique, dans un environnement acide.

16. Procédé selon la revendication 1, comprenant par ailleurs la préparation de triacétyl aloé-émodine par conversion d'aloïne en aloé-émodine par traitement avec un sel ferrique dans un environnement acide avec ensuite acétylation avec de l'anhydride acétique en présence d'acide sulfurique comme catalyseur, 'avec ensuite cristallisation de la triacétyl aloé-émodine en utilisant du toluène, éventuellement en mélange avec du méthanol.
